# EUROPÄISCHE PATENTSCHRIFT

(11) **EP 1 475 157 B1**
(45) Veröffentlichungstag und Bekanntmachung des Hinweises auf die Patenterteilung: **05.01.2011**
(21) Anmeldenummer: 04010038.0
(22) Anmeldetag: 28.04.2004
(51) Int. Cl.: B05B 1/14, A61M 15/08

(54) **Düse für ein Nasenspray mit mehreren Öffnungen zum Sprühen von viskosem Material**
Nozzle for nasal sprayer having a plurality of exit orifices for spraying viscous material
Buse d'un pulvérisateur nasal comprenant plusieurs orifices de sortie pour délivrer des substances visqueuses

(30) Priorität: 06.05.2003 DE 10321902
(43) Veröffentlichungstag der Anmeldung: 10.11.2004
(73) Patentinhaber: Ing. Erich Pfeiffer GmbH, 78315 Radolfzell (DE)
(72) Erfinder: Merk, Hans, 78343 Horn (DE); Krampen, Gerald, 78315 Radolfzell (DE); Ritsche, Stefan, 78253 Eigeltingen (DE)
(74) Vertreter: Patentanwälte Ruff, Wilhelm, Beier, Dauster & Partner

(56) Entgegenhaltungen:
- EP-A- 0 494 803
- WO-A-99/32185
- FR-A- 2 467 604
- US-A- 5 358 179

## Beschreibung

Die Erfindung betrifft eine Austragvorrichtung für zumindest ein Medium mit einem Mediumspeicher, einer Mediumpumpe und einem Applikator, in dem eine Mediumführung vorgesehen ist und der einen dem Mediumspeicher abgewandten und an einem schlanken Endbereich angebrachten Austrittsbereich für einen Mediumaustrag aufweist.

Austragvorrichtungen für zumindest ein Medium mit einem Mediumspeicher, einer Mediumpumpe und einem Applikator sind in vielfältigen Ausführungen aus dem Stand der Technik bekannt. Die EP494803 zeigt eine Austragvorrichtung mit den im Oberbegriff des Anspruchs 1 genannten Merkmalen. In der EP 1 219 356 A1 wird eine Austragvorrichtung beschrieben, bei der ein auszutragendes Medium vor dem Austrag in einem Mediumspeicher aufbewahrt wird, der als Glasbehälter ausgeführt ist. Der Mediumspeicher wird durch eine elastische Dichtung verschlossen, die bei manueller Betätigung der Austragvorrichtung durch einen Benutzer als Schubkolben einer Mediumpumpe wirksam wird. Bei Aktivierung der Mediumpumpe wird das in dem Mediumspeicher bevorratete Medium unter Druck gesetzt und in einen Applikator gepresst. An einem dem Mediumspeicher abgewandten, schlanken Endbereich des Applikators ist eine Austragdüse vorgesehen, durch die das Medium in eine Umgebung abgegeben werden kann. Dabei kann insbesondere eine kegelförmige Zerstäubung des auszutragenden Mediums vorgesehen werden. In dem Applikator ist ein Füllstück vorgesehen, das zur Medienführung von der Mediumpumpe hin zu der Austrittsdüse dient und bei in Spritzgusstechnik hergestellten Kunststoffapplikatoren aus produktionstechnischen Gründen notwendig ist. Das Füllstück weist abschnittsweise eine nahezu zylindrische Form auf und enthält in zentrischer Anordnung einen Mediumkanal zur Leitung des Mediums. Anwendung finden bekannte Austragvorrichtungen insbesondere bei der Verabreichung von niedrigviskosen, wässrigen Medikamenten, wobei ein einmaliger oder mehrfacher Austrag des Mediums aus der Austragvorrichtung vorgesehen sein kann.

Die Aufgabe der Erfindung ist es, eine Austragvorrichtung der eingangs genannten Art zu schaffen, die für einen gezielten Austrag insbesondere hochviskoser oder gelartiger Medien geeignet ist.

Diese Aufgabe wird dadurch gelöst, dass an dem Austrittsbereich zumindest zwei Austrittsöffnungen vorgesehen sind. Bei einer manuell zu betätigenden Austragvorrichtung lässt sich mit vertretbarem Aufwand kein derartig hoher Druck erzeugen, der die Zerstäubung eines insbesondere hochviskosen oder gelartigen Mediums erlauben würde. Um dennoch einen Mediumaustrag ähnlich wie bei niedrigviskosen, wässrigen Medien für ein insbesondere hochviskoses oder gelartiges Medium zu simulieren, sind an dem Austrittsbereich zumindest zwei Austrittsöffnungen vorgesehen. Durch diese Austrittsöffnungen tritt das Medium als paralleler oder leicht divergierender Mediumstrahl aus. Damit ergibt sich für den Benutzer ein zumindest ähnliches Austragbild wie bei einer aus dem Stand der Technik bekannten Austragvorrichtung für niedrigviskose, insbesondere wässrige Medien. Unterstützend können an den Austrittsöffnungen Vorkehrungen vorgesehen sein, die eine Strahlaufweitung begünstigen. Zu nennen sind hierfür insbesondere unmittelbar nach der Austrittsöffnung angeordnete Leitelemente wie Streugitter oder Leitschaufeln. Bei einer Verwendung der erfindungsgemäßen Austragvorrichtung für niedrigviskose, insbesondere wässrige Medien lässt sich durch die zumindest zwei Austrittsöffnungen an dem Austrittsbereich eine besonders vorteilhafte Verteilung des auszutragenden Mediums erzielen. Die zumindest zwei Austrittsöffnungen werden dazu mit einem deutlich reduzierten Austrittsquerschnitt gegenüber üblichen Austrittsöffnungen versehen, wodurch sich für jede Austrittsöffnung ein besonders feiner, insbesondere vernebelter Mediumstrahl ergibt. Dabei kann dennoch mit einem Austraghub der Austragvorrichtung eine zumindest weitgehend ähnliche Austragscharakteristik des auszutragenden Mediums wie bei bekannten Austragvorrichtungen gefördert werden. Vorzugsweise weist die Mediumführung ein in dem Applikator angeordnetes Füllstück auf, das als separates Teil, als der Mediumpumpe zugeordnetes Teil oder als mit dem Applikator verbundenes Teil ausgeführt sein kann.

In Ausgestaltung der Erfindung sind die Austrittsöffnungen für den Mediumaustrag in zumindest zwei unterschiedliche Hauptaustragrichtungen ausgerichtet. Eine Hauptaustragrichtung einer Austrittsöffnung kann durch eine Mittelung aller aus dieser Austrittsöffnung bei einem Mediumaustrag auftretenden Mediumvektoren ermittelt werden. Dabei wird jedem ausgetragenen Mediumteilchen ein Richtungsvektor und in Abhängigkeit von seinem Volumen ein proportionaler Gewichtungsfaktor zugeordnet, wodurch sich der jeweilige Mediumvektor ergibt. Durch Addition aller Mediumvektoren kann eine Hauptaustragrichtung für die entsprechende Austrittsöffnung bestimmt werden. Eine unterschiedliche Ausrichtung der zumindest zwei Hauptaustragrichtungen kann sowohl in einer gemeinsamen Ebene der Hauptaustragrichtungen als auch durch eine windschiefe Anordnung der Hauptaustragrichtungen verwirklicht werden. Bei einer Mehrzahl von unterschiedlichen Hauptaustragrichtungen sind auch Kombinationen von parallelen, in gleichen Ebenen angeordneten und zueinander windschief vorgesehenen Hauptaustragrichtungen denkbar. Damit kann eine gezielte Benetzung nebeneinander oder gegenüberliegend angeordneter Applikationsbereiche, für die das auszutragende Medium vorgesehen ist, erreicht werden.

In weiterer Ausgestaltung der Erfindung ist zumindest eine Austrittsöffnung für eine Hauptaustragrichtung in einem Winkelbereich von 10° bis 80° zu einer Applikatormittelachse angeordnet. Eine Applikatormittelachse wird durch eine Außengeometrie des Applikators bestimmt. Der erfindungsgemäße Applikator erstreckt sich in einer Haupterstreckungsrichtung und ist mit dem schlanken Endbereich versehen. Die Applikatormittelachse entspricht im wesentlichen der Haupterstreckungsrichtung des Applikators und kennzeichnet damit gleichzeitig zumindest nahezu eine Hauptflussrichtung des auszutragenden Mediums im Applikator. Durch eine Anordnung zumindest einer Austrittsöffnung in einem Winkelbereich von 10° bis 80° zu einer Applikatormittelachse lässt sich insbesondere bei einem Austrag des Mediums in natürliche Körperöffnungen wie Nase, Ohren oder Mund eine gezielte Applikation des Mediums auf bestimmte Bereiche innerhalb der Körperöffnungen erreichen.

In weiterer Ausgestaltung der Erfindung sind die zumindest zwei Austrittsöffnungen über einen Umfang des Applikators gleichmäßig verteilt zu der Applikatormittelachse angeordnet. Durch eine gleichmäßige Verteilung der zumindest zwei Austrittsöffnungen über einen Umfang des Applikators kann nahezu unabhängig von einer Ausrichtung des Applikators durch den Benutzer eine Benetzung der mit dem Medium zu beaufschlagenden Bereiche, insbesondere innerhalb von Körperöffnungen, sichergestellt werden. Eine gleichmäßige Verteilung der Austrittsöffnungen liegt insbesondere dann vor, wenn die Hauptaustragrichtungen der Austrittsöffnungen auf einer rotationssymmetrischen Geometrie gleichmäßig voneinander beabstandet in radial gleichen Winkeln zueinander angeordnet sind. Dabei kann die rotationssymmetrische Geometrie insbesondere zylindrisch, sphärisch oder kegelförmig ausgestaltet sein.

In weiterer Ausgestaltung der Erfindung ist in Austragrichtung gesehen vor zumindest einer Austrittsöffnung eine Strömungsleitgeometrie für das Medium vorgesehen. Eine Strömungsleitgeometrie wird insbesondere durch eine Wechselwirkung einer Innengeometrie des Applikators mit einer Geometrie des Füllstücks erzielt. Durch die Strömungsleitgeometrie kann einerseits ein Einfluss auf die Hauptaustragrichtung des auszutragenden Mediums genommen werden, andererseits ist auch eine Beeinflussung eines Austrittsverhaltens des auszutragenden Mediums möglich. Dies wird insbesondere durch eine Umlenkung des Mediums vor zumindest einer Austrittsöffnung erreicht, wodurch das auszutragende Medium zusätzliche Impulse erhält, die Auswirkungen auf ein Strahlverhalten nach Austritt aus der Austrittsöffnung haben. Geeignete Strömungsleitgeometrien sind insbesondere Drallkanäle, scharfe oder verrundete Kanten oder Hinterschnitte zur Erzeugung von Verwirbelungen.

In weiterer Ausgestaltung der Erfindung weist ein Mediumkanal des Füllstücks an einer dem Austrittsbereich zugewandten Stirnseite eine stirnseitige, konische Vertiefung als Strömungsleitgeometrie auf. Damit kann insbesondere bei einem mit einer Innenbohrung versehenen und als Hülse ausgeführten Füllstück eine Aufweitung und damit eine Geschwindigkeitserhöhung eines Mediumstroms im Füllstück erzielt werden, was einer Beeinflussung des Medienstrahls dienlich ist. Die dazu vorgesehene konische Vertiefung weitet sich dabei in Fließrichtung des Mediums auf und nimmt an der Stirnseite des Füllstücks einen maximalen Querschnitt ein.

In weiterer Ausgestaltung der Erfindung ist an einer Innengeometrie des Applikators wenigstens ein Distanzelement für die Positionierung des Füllstücks vorgesehen. Mit Hilfe eines Distanzelementes ist eine einfache und kostengünstige sowie präzise Montage des Füllstücks in den Applikator möglich, wobei das Distanzelement für eine definierte Positionierung des Füllstücks im Applikator und damit für einen präzisen Mediumkanal sorgt. Da sich Maßabweichungen in dem Mediumkanal stark auf Austrittseigenschaften des Mediums auswirken, besteht hier ein starkes Interesse an einer präzisen und dennoch einfach zu verwirklichenden Beziehung zwischen Applikator und Füllstück. Das Distanzelement ist dabei insbesondere als formschlüssig wirkende Anschlagkante im Applikator vorgesehen, gegen die das Füllstück während der Montage angepresst und gegebenenfalls ergänzend durch Kraft-, Formoder Stoffschluss fixiert wird.

In weiterer Ausgestaltung der Erfindung ist an einer der Stirnseite des Füllstücks zugewandten Innenfläche des Applikators ein Führungskonus als Strömungsleitgeometrie vorgesehen. Eine Mittelachse des Führungskonus ist dabei insbesondere nahezu parallel zur Applikatormittelachse angeordnet und weitet einen nahezu parallel zu diesen Achsen auftreffenden Mediumstrahl gleichförmig auf, so dass eine radiale Verteilung des Mediums auf zumindest zwei Austrittsöffnungen erleichtert wird. In einer bevorzugten Ausführungsform ist der Führungskonus als Kegelstumpf ausgeführt, so dass das anströmende Medium zuerst an einer Kegelstumpfstirnfläche auftritt und dann entlang des sich aufweitenden Kegelstumpfes in Richtung der Austrittsöffnungen weitergeleitet wird. In einer besonders bevorzugten Ausführungsform ist der Führungskonus des Applikators mit einer konischen Vertiefung des Füllstücks kombiniert, so dass sich ein durchgängig gleicher oder in Richtung der Austrittsöffnung aufweitender Querschnitt für das Medium ergibt, wodurch eine laminare oder turbulente Strömung des Mediums erzielbar ist.

In weiterer Ausgestaltung der Erfindung ist in dem Applikator für zumindest eine Austrittsöffnung eine Ventileinrichtung vorgesehen. Eine Ventileinrichtung ermöglicht eine Steuerung eines Medienaustrags aus einer Austrittsöffnung insbesondere durch Öffnen bzw. teilweises oder vollständiges Verschließen der Austrittsöffnung oder eines entsprechend vor der Austrittsöffnung angeordneten und durch die Ventileinrichtung beeinflussbaren Medienkanals. Als Ventileinrichtungen kommen insbesondere druckgesteuerte Ventile oder mechanisch angesteuerte Ventile in Frage. Mit Hilfe einer Ventileinrichtung kann insbesondere eine Austrittsgeschwindigkeit des Mediums über einen Austragvorgang auf einem durch die Viskosität des Mediums bestimmten Mindestniveau gehalten werden. Dabei kann insbesondere zu Beginn und am Ende des Austragvorganges zumindest eine Austrittsöffnung durch die Ventileinrichtung verschlossen werden, um die Mindestgeschwindigkeit des Mediums sicherzustellen.

In weiterer Ausgestaltung der Erfindung ist die Ventileinrichtung durch eine Steuereinrichtung ansteuerbar vorgesehen. Eine Steuereinrichtung ermöglicht ein gezieltes Ansprechen einer Ventileinrichtung, insbesondere über mechanische, elektromagnetische oder elektrische Stelleinrichtungen. Die Steuereinrichtung nimmt dabei insbesondere Einfluss auf zumindest eine Ventileinrichtung zum zumindest zeitweiligen Öffnen und Verschließen zumindest einer Austrittsöffnung in Abhängigkeit des Austragvorganges, wobei insbesondere zu Beginn und am Ende des Ausgangvorganges zumindest eine Austrittsöffnung zur Aufrechterhaltung eines Mindestdrucks verschlossen werden kann und/oder durch wechselndes Öffnen und Schließen von Austrittsöffnungen über zugeordnete Ventileinrichtungen ein alternierender, insbesondere umlaufender Mediumstrahl erzielbar ist.

In weiterer Ausgestaltung der Erfindung sind die zumindest zwei Austrittsöffnungen derart relativ zueinander ausgerichtet, dass sich ihre Hauptaustragrichtungen kreuzen. Sofern sich die Hauptaustragrichtungen austretender Mediumstrahlen kreuzen, kommt es zu einer Wechselwirkung der Mediumstrahlen und damit zu einer Beeinflussung eines Strahlbildes, das von der Austragvorrichtung während des Medienaustrags erzielt wird. Damit kann eine Konzentrationsverteilung des austretenden Mediums vorherbestimmt werden, die insbesondere auf Erfordernisse einer Mediumdosierung in einem Anwendungsbereich des Mediums angepasst ist. Insbesondere bei hochviskosen, gelartigen Medien, die beim Austrag nicht zerstäubt werden, kann durch die Kreuzung der Austragsrichtungen die gewünschte Konzentrationsverteilung des austretenden Mediums erzielt werden.

In weiterer Ausgestaltung der Erfindung ist der Applikator als einstückiges Kunststoffbauteil hergestellt. Dadurch kann eine vorteilhafte und kostengünstige Herstellung des Applikators erzielt werden, Montagevorgänge, wie sie insbesondere für die Anbringung eines Füllstücks notwendig wären, können dadurch entfallen.

In weiterer Ausgestaltung der Erfindung ist in den Applikator zumindest ein Mediumkanal einstückig integriert. Dadurch ist eine Strömungsgeometrie, die für den Austrag des Mediums aus den Austrittsöffnungen erheblich ist, vollständig durch den Applikator definiert. Eine toleranzbehaftete Wechselwirkung des Applikators mit anderen Komponenten einer Mediumpumpe im Bereich der Austrittsöffnungen kann dadurch vermieden werden.

In weiterer Ausgestaltung der Erfindung weist der Mediumkanal einen mit einer Mediumpumpe verbindbaren Eintrittsbereich auf, der derart gestaltet ist, dass er in montiertem Zustand mit einem Austrittsstutzen der Mediumpumpe eine dichte Steckverbindung eingeht. Damit wird eine vorteilhafte Montage des Applikators auf die Mediumpumpe ermöglicht, bei der in einem Arbeitsschritt eine dichtende Wirkverbindung, die auch zur Übertragung von Betätigungskräften geeignet ist, hergestellt wird.

In weiterer Ausgestaltung der Erfindung ist der Mediumkanal in einem der Mediumpumpe abgewandten Austrittsbereich in einzelne, vom Mediumkanal auf die Austrittsöffnungen zulaufende Kanalabschnitte aufgetrennt. Damit wird eine Ausrichtung des Mediums auf die entsprechende Austrittsöffnung gewährleistet, wodurch ein vorteilhafter Austragvorgang unterstützt wird. Durch die Kanalabschnitte, die nur eine abschnittsweise Auftrennung eines Stirnbereiches des Applikators bewirken, wird eine stabile Verbindung des von den Austrittsöffnungen im wesentlichen umschlossenen Stirnbereichs mit anderen Applikatorabschnitten gewährleistet.

In weiterer Ausgestaltung der Erfindung ist der Mediumkanal mit einem im wesentlichen konstanten oder in Austragrichtung gleichmäßig verjüngten Querschnitt ausgebildet. Dadurch wird eine vorteilhafte Herstellbarkeit des Mediumkanals im Spritzgussverfahren gewährleistet.

In weiterer Ausgestaltung der Erfindung weist der Mediumkanal einen im wesentlichen sternförmigen Querschnitt auf. Damit nimmt der Mediumkanal nur ein geringes Volumen ein und kann dennoch eine vorteilhafte Mediumversorgung aller Austrittsöffnungen gewährleisten. Durch den sternförmigen Querschnitt wird auch eine vorteilhafte Stabilisierung des Applikators erreicht.

Weitere Vorteile und Merkmale ergeben sich aus den Ansprüchen sowie der Beschreibung bevorzugter Ausführungsbeispiele der Erfindung, die anhand der Zeichnungen dargestellt sind.
- Fig. 1: zeigt in isometrischer Darstellung einen Applikator für eine Aus- tragvorrichtung,
- Fig. 2: in ebener Schnittdarstellung einen Applikator vor Montage eines Füllstücks,
- Fig. 3: eine Ansicht von unten eines Applikators vor Montage eines Füllstücks,
- Fig. 4: in ebener Schnittdarstellung einen Endbereich eines Applikators mit montiertem Füllstück,
- Fig. 5: in ebener Schnittdarstellung den Endbereich des Applikators mit montiertem Füllstück,
- Fig. 6: in ebener Schnittdarstellung einen Querschnitt einer weiteren Ausführungsform eines Applikators mit montiertem Füllstück und
- Fig. 7: in ebener Schnittdarstellung einen Querschnitt durch eine weite- re Ausführungsform eines Applikators mit montiertem Füllstück,
- Fig. 8: in ebener Schnittdarstellung einen als einstückiges Kunststoffteil ausgeführten Applikator
- Fig. 9: in ebener Schnittdarstellung einen Querschnitt des Applikators gemäß der Fig. 8.

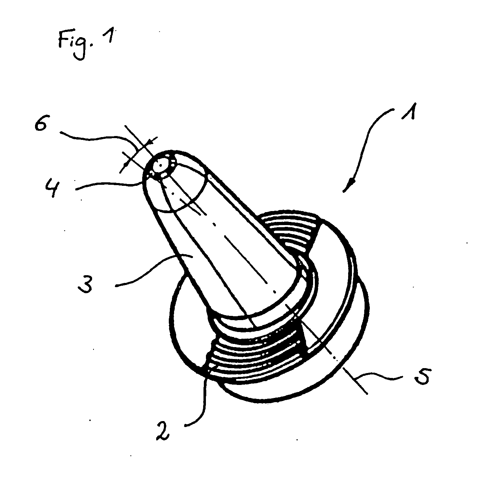

Alle in den Fig. 1 bis 9 dargestellten Ausführungen eines Applikators sind zur Verwendung mit einer nicht näher dargestellten Austragvorrichtung vorgesehen, wobei die Austragvorrichtung einen Mediumspeicher, der insbesondere als ein- oder mehrteiliger Behälter aus Glas, Kunststoff oder Metall ausgeführt ist, sowie eine Mediumpumpe, die insbesondere als Schubkolbenpumpe ausgeführt sein kann, aufweist. An der Mediumpumpe wird an einer dem Mediumspeicher abgewandten Seite ein Applikator 1, vorliegend in Form einer Nasenolive, angebracht, der zur Weiterleitung des von der Mediumpumpe aus dem Mediumbehälter ausgetragenen und unter Druck gestellten Mediums bestimmt ist. Der Applikator 1 weist für eine manuelle Betätigung der Mediumpumpe zwei Fingerauflageflächen 2 auf, auf die ein Benutzer zur Betätigung der Austragvorrichtung insbesondere Zeige- und Mittelfinger auflegen kann, während der Daumen des Benutzers auf eine der Fingerauflagefläche 2 abgewandte und nicht näher dargestellte Unterseite der Austragvorrichtung aufgelegt wird. Weiterhin weist eine Außenkontur des Applikators 1 einen dem nicht näher dargestellten Mediumspeicher abgewandten, schlanken Endbereich 3 auf, der bei dem vorliegenden Ausführungsbeispiel konisch verjüngt und an einer Stirnseite sphärisch verrundet vorgesehen ist. An dem Endbereich sind stirnseitig mehrere Austrittsöffnungen 4 vorgesehen, die gleichmäßig verteilt zirkular auf einem einheitlichen Radius des Endbereiches angeordnet sind. Die Austrittsöffnungen 4 weisen einen im wesentlichen rechteckigen Querschnitt auf und sind gegenüber einer Applikatormittelachse 5 um einen Neigungswinkel 6 gekippt. Dadurch kann mit Hilfe des Applikators 1 ein nicht näher dargestelltes Strahlbild beim Austrag eines Mediums erzielt werden, bei dem Mediumstrahlen durch die Austrittsöffnungen jeweils unter dem Neigungswinkel 6 zur Applikatormittelachse 5 voneinander beabstandet austreten.

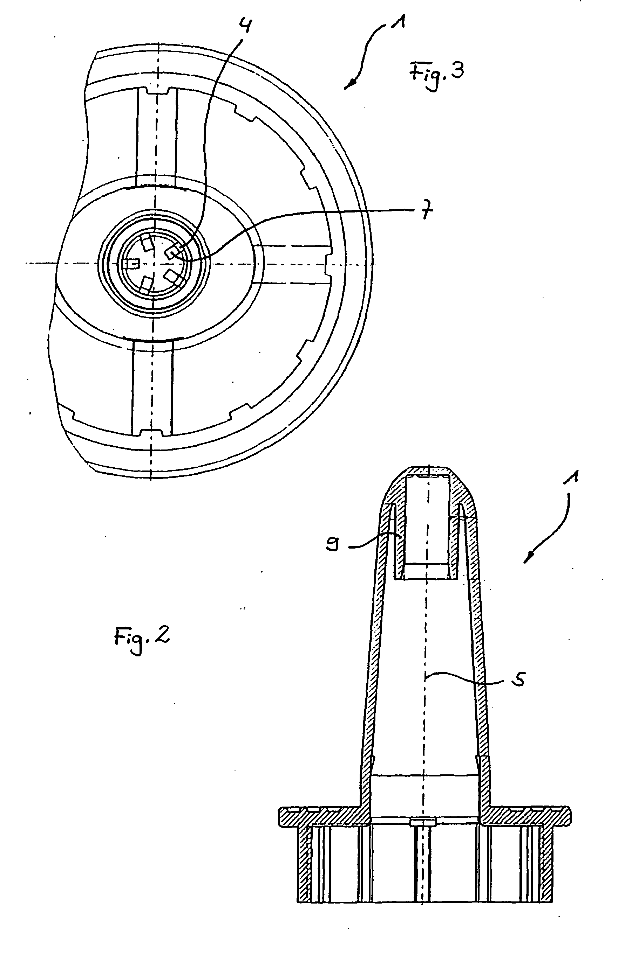

Dazu sind bei einer in den Fig. 2 und 3 dargestellten Ausführungsform des Applikators für eine Austragvorrichtung zirkular auf einem Umfang angeordnete und radial ausgerichtete Austrittskanäle 7 vorgesehen, durch die das Medium den Austrittsöffnungen 4 zugeführt wird. Zur Montage eines in den Fig. 2 und 3 nicht näher dargestellten Füllstückes 8 ist an dem Applikator 1 eine in Fig. 2 dargestellte Führungsbuchse 9 vorgesehen, die eine im wesentlichen zylindrische Bohrung aufweist. In diese Führungsbuchse 9 ist das Füllstück kraftschlüssig einpressbar vorgesehen und kann bei Bedarf stoffschlüssig befestigt werden.

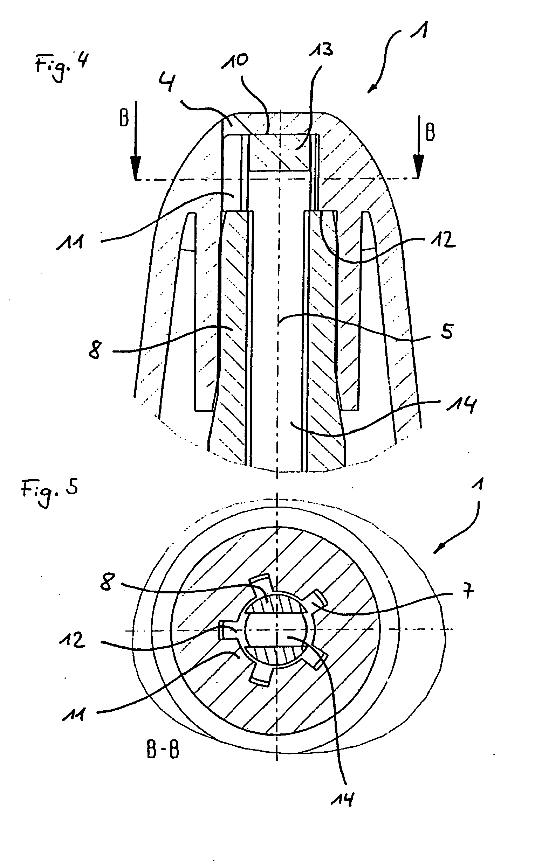

In einer weiteren Ausführungsform des Applikators, die in der Fig. 4 dargestellt ist, erstrecken sich von einem Innenboden 10 der Führungsbuchse 9 parallel zur Applikatormittelachse 5 mehrere als Distanzrippen 11 ausgeführte Distanzelemente, an deren dem Innenboden 10 abgewandter Stirnseite eine Stirnfläche 12 des Füllstückes 8 formschlüssig anliegt. Das Füllstück 8 ist bei der in Fig. 4 dargestellten Ausführungsform des Applikators mit einem U-förmigen Bügelfortsatz 13 versehen, der mit beiden Schenkeln des U auf der Stirnfläche des Füllstückes angebracht ist, während eine Querstrebe des U parallel zum Innenboden 10 des Applikators 1 verläuft. In Zusammenwirkung mit den Distanzrippen 11 bildet somit das Füllstück 8 eine Strömungsleitgeometrie, die in Zusammenwirkung mit den Austrittsöffnungen 4 für einen definierten Austrag des Mediums aus dem Applikator verantwortlich sind. Die Zufuhr des auszutragenden Mediums zu der Strömungsleitgeometrie erfolgt über einen Mediumkanal 14, der als zentrale Bohrung im Füllstück 8 vorgesehen ist.

Die Fig. 5 gibt die in Fig. 4 dargestellten Geometrien in einer Schnittebene orthogonal zur Applikatormittelachse 5 wieder. Dabei sind die durch das Füllstück 8 und den Applikator 1 gebildeten Austrittskanäle 7 zwischen den Distanzrippen gut zu erkennen. Weiterhin ist in Fig. 5 der Mediumkanal 14 als zylindrische Bohrung im Füllstück 8 zu erkennen, während die beiden Schenkel des U-förmigen Bügelfortsatzes 13 in geschnittener Darstellung gezeigt werden.

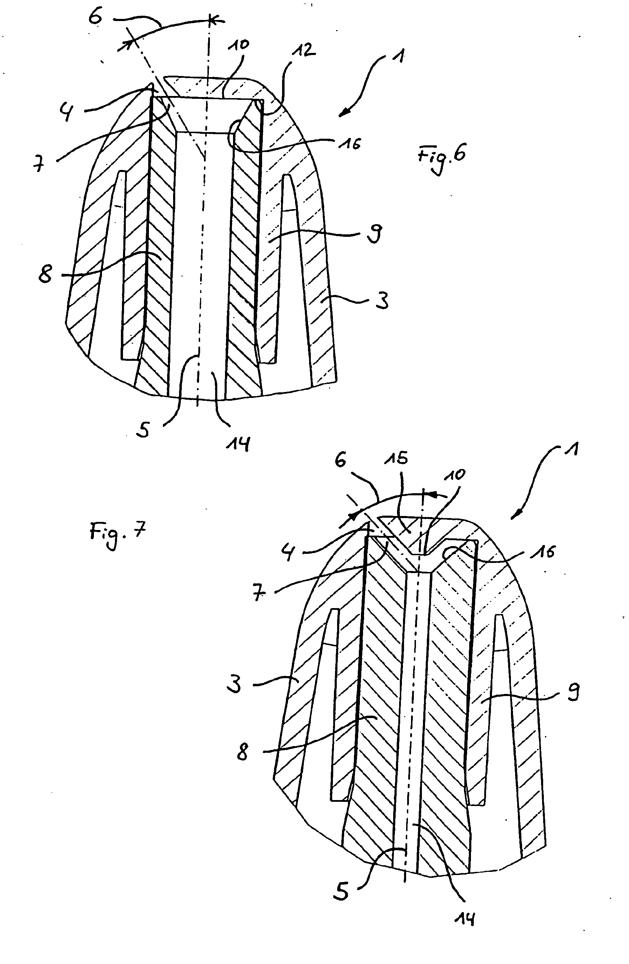

Bei einer weiteren Ausführungsform des Applikators für eine Austragvorrichtung nach Fig. 6 ragt das Füllstück 8 bis unmittelbar vor die Austrittsöffnung 4 in die Führungsbuchse 9 des Applikators 1 hinein und bildet mittels eines stirnseitig am Füllstück 8 vorgesehenen und sich zur Austrittsöffung 4 hin erweiternden Austrittskonus 16 eine Strömungsleitgeometie für das austretende Medium. Der Innenboden 10 des Applikators ist bei dieser Ausführungsform eben gestaltet. Der Neigungswinkel 6 der Hauptaustragrichtung wird im wesentlichen durch den Austrittsköffung 6 in Kombination mit der Geometrie der Austrittsöffnung 4 bestimmt.

Bei einer in Fig. 7 dargestellten weiteren Ausführungsform eines Applikators für eine Austragvorrichtung ist der Innenboden 10 des Applikators 1 abschnittsweise als kegelstumpfförmiger Führungskonus 15 ausgeführt, der in einen Austrittskonus 16 des Füllstücks 8 hineinragt und somit in Zusammenwirkung mit dem Austrittskonus 16 und der Geometrie der Austrittsöffnung 4 den Austrittskanal 7 bildet. Der Führungskonus 15 und der Austrittskonus 16 stellen eine Strömungsleitgeometrie im Sinne der Erfindung dar.

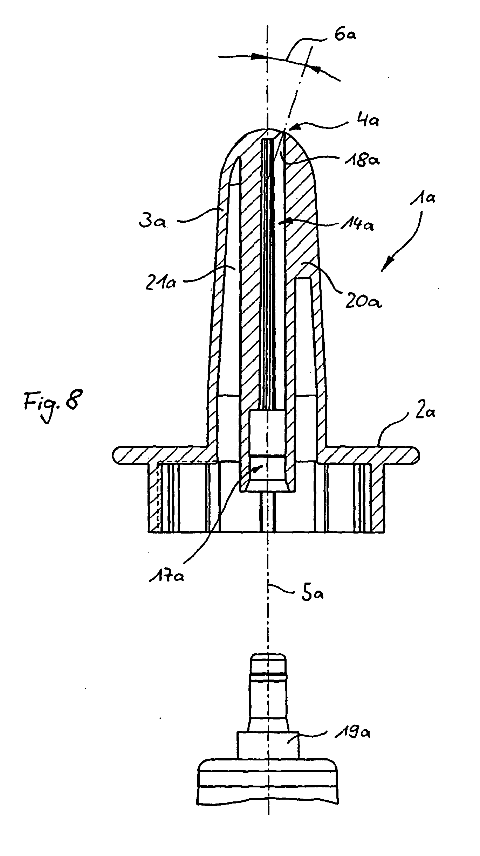

Bei einer in Fig. 8 dargestellten, weiteren Ausführungsform eines Applikators für eine Austragvorrichtung sind, analog der Darstellung in Fig. 2, fünf gleichmäßig über den Umfang verteilte Austrittsöffnungen 4a vorgesehen. Der Applikator 1a ist als einstückiges Kunststoffspritzgussteil hergestellt und weist einen einstückig ausgeführten Mediumkanal 14a auf. Aufgrund der in Fig. 8 gewählten Schnittebene und der Verteilung der Austrittsöffnungen 4a über den Umfang des Applikators 1a ist nur ein Teilbereich des Mediumkanals 14a in der Schnittdarstellung gemäß der Fig. 8 sichtbar. Der Mediumkanal 14a wird bei der Herstellung des Applikators 1a im Spritzgussverfahren insbesondere durch einen schlanken, in einer Spritzgussform vorgesehenen Kern freigehalten.

An einem dem Austrittsbereich abgewandten Eintrittsbereich 17a ist ein Querschnitt des Mediumkanals 14a derart erweitert, dass ein in Fig. 8 schematisch dargestellter Austrittsstutzen 19a einer nicht näher dargestellten Mediumpumpe form- und/oder kraftschlüssig angekoppelt werden kann. Der Mediumkanal 14a ist in dem der Mediumpumpe abgewandten Austrittsbereich in einzelne, vom Mediumkanal 14a auf die Austrittsöffnungen 4a zulaufende Kanalabschnitte 18a aufgetrennt und erlaubt damit eine gleichmäßige Verteilung des auszutragenden Mediums auf die einzelnen Austrittsöffnungen 4a.

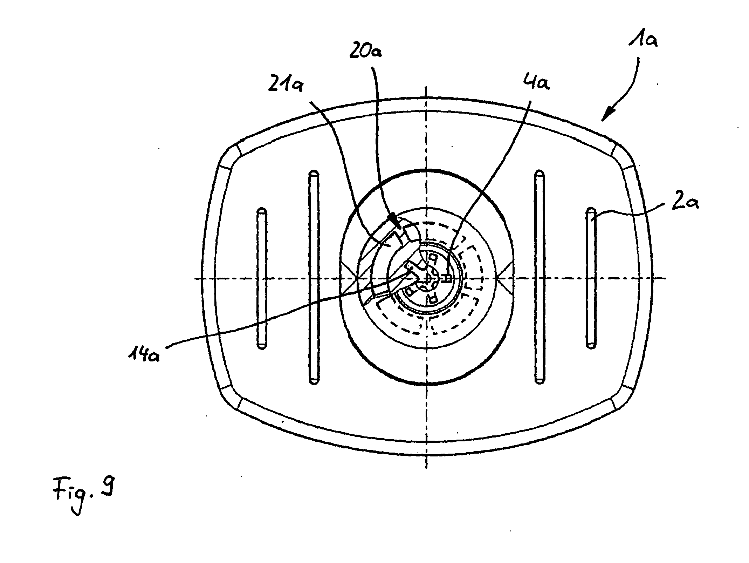

Der Mediumkanal 14a weist einen, in Fig. 9 näher dargestellten, im wesentlichen konstanten Querschnitt auf, der für eine vorteilhafte Herstellung im Kunststoffspritzgussverfahren mit einer gleichmäßigen Verjüngung in Richtung der Austrittsöffnungen 4a versehen ist, um ein Entformen aus der Spritzgussform zu erleichtern. Der Querschnitt des Mediumkanals 14a ist im wesentlichen sternförmig gestaltet, so dass der Kern der Spritzgussform trotz der schlanken Ausführung eine vorteilhafte Stabilität aufweist und der Mediumkanal ein geringes Gesamtvolumen aufweist. Durch das geringe Gesamtvolumen kann verhindert werden, das eine erhebliche Menge des auszutragenden Mediums in dem Applikator 1a verbleibt und somit ein unerwünschtes Nachtropfen nach Durchführung der Mediumdosierung stattfinden kann. Durch den sternförmigen Querschnitt des Mediumkanals 14a wird auch eine vorteilhafte Stabilität des Applikators 1a bewirkt. Die Sternform wird im Querschnitt bei der dargestellten Ausführung durch fünf Sternzacken gebildet, die axial etwa mit den fünf Austrittsöffnungen 4a fluchten.

Die Stabilität des Applikators 1a wird auch durch Versteifungsstege 20a unterstützt, die sich in radialer Richtung von der Applikatormittelachse 5 erstrecken und den Mediumkanal 14a im Applikator 1a abstützen. In Bereichen abseits der sternförmigen Ausläufer des Mediumkanals 4a sind Hohlräume 21a vorgesehen, die ebenfalls wie der Mediumkanal 4a durch Kerne in der Spritzgussform verwirklicht werden und zur Reduzierung von Materialanhäufungen für die als Kunststoffspritzgussteil ausgeführten Applikator 1a vorgesehen sind. Entsprechende Materialanhäufungen können zu einer Verschlechterung einer Oberflächenqualität, beispielsweise durch Schrumpfungs- und Einfallstellen führen.

## Patentansprüche

1. Austragvorrichtung für zumindest ein Medium mit einem Mediumspeicher, einer Mediumpumpe und einem in Richtung einer Applikatormittelachse ausgerichteten Applikator (1, 1a) in Form einer Nasenolive, in dem eine Mediumführung vorgesehen ist und der einen dem Mediumspeicher abgewandten und an einem schlanken Endbereich angebrachten Austrittsbereich für einen Mediumaustrag aufweist, wobei an dem Austrittsbereich zumindest zwei Austrittsöffnungen (4, 4a) vorgesehen sind, **dadurch gekennzeichnet, dass** die Austrittsöffnungen (4, 4a) jeweils eine radial außenliegende Wandung aufweisen, die parallel zur Applikatormittelachse ausgerichtet ist, und jeweils eine radial innenliegende Wandung aufweisen, die gegenüber der Applikatormittelachse geneigt ist.

2. Austragvorrichtung nach Anspruch 1, **dadurch gekennzeichnet, dass** die Austrittsöffnungen (4, 4a) für den Mediumaustrag in zumindest zwei unterschiedliche Hauptaustragrichtungen ausgerichtet sind.

3. Austragvorrichtung nach Anspruch 1 oder 2, **dadurch gekennzeichnet, dass** zumindest eine Austrittsöffnung für eine Hauptaustragrichtung in einem Winkelbereich (6, 6a) von 10° bis 80° zu der Applikatormittelachse angeordnet ist.

4. Austragvorrichtung nach einem der vorhergehenden Ansprüche, **dadurch gekennzeichnet, dass** die zumindest zwei Austrittsöffnungen (4, 4a) über einen Umfang des Applikators (1, 1a) gleichmäßig verteilt zu einer Applikatormittelachse (5, 5a) angeordnet sind.

5. Austragvorrichtung einem der vorhergehenden Ansprüche, **dadurch gekennzeichnet, dass** in Austragrichtung gesehen vor zumindest einer Austrittsöffnung (4) eine Strömungsleitgeometrie (15, 16) für das Medium vorgesehen ist.

6. Austragvorrichtung nach Anspruch 5, **dadurch gekennzeichnet, dass** ein Mediumkanal (14) eines Füllstücks (8) an einer dem Austrittsbereich zugewandten Stirnseite eine stirnseitige, konische Vertiefung als Strömungsleitgeometrie (15, 16) aufweist.

7. Austragvorrichtung einem der vorhergehenden Ansprüche, **dadurch gekennzeichnet, dass** an einer Innengeometrie des Applikators (1) wenigstens ein Distanzelement (11) für die Positionierung eines Füllstücks (8) vorgesehen ist.

8. Austragvorrichtung nach Anspruch 5, **dadurch gekennzeichnet, dass** an einer der Mediumpumpe zugewandten Innenfläche (10) des Applikators ein Führungskonus (15) als Strömungsleitgeometrie (15, 16) vorgesehen ist.

9. Austragvorrichtung nach einem der vorhergehenden Ansprüche, **dadurch gekennzeichnet, dass** in dem Applikator (1, 1a) für zumindest eine Austrittsöffnung (4, 4a) eine Ventileinrichtung vorgesehen ist.

10. Austragvorrichtung nach Anspruch 9, **dadurch gekennzeichnet, dass** die Ventileinrichtung durch eine Steuereinrichtung ansteuerbar vorgesehen ist.

11. Austragvorrichtung nach einem der vorhergehenden Ansprüche, **dadurch gekennzeichnet, dass** die zumindest zwei Austrittsöffnungen (4, 4a) derart relativ zueinander ausgerichtet sind, dass sich ihre Hauptaustragrichtungen kreuzen.

12. Austragvorrichtung nach einem der Ansprüche 1 bis 5 bzw. 8 bis 11, **dadurch gekennzeichnet, dass** der Applikator (1a) als einstückiges Kunststoffbauteil hergestellt ist.

13. Austragvorrichtung nach einem der Ansprüche 1 bis 5 bzw. 8 bis 12, **dadurch gekennzeichnet, dass** in den Applikator (1a) zumindest ein Mediumkanal (14a) einstückig integriert ist.

14. Austragvorrichtung nach Anspruch 13, **dadurch gekennzeichnet, dass** der Mediumkanal (14a) einen mit einer Mediumpumpe verbindbaren Eintrittsbereich (17a) aufweist, der derart gestaltet ist, dass er in montiertem Zustand mit einem Austrittsstutzen (19a) der Mediumpumpe eine dichte Steckverbindung eingeht.

15. Austragvorrichtung nach Anspruch 13 oder 14, **dadurch gekennzeichnet, dass** der Mediumkanal (14a) in einem der Mediumpumpe abgewandten Austrittsbereich in einzelne, auf die Austrittsöffnungen (4a) zulaufende Kanalabschnitte (18a) aufgetrennt ist.

16. Austragvorrichtung nach Anspruch 13, **dadurch gekennzeichnet, dass** der Mediumkanal (14a) mit einem im wesentlichen konstanten oder in Austragrichtung gleichmäßig verjüngten Querschnitt ausgebildet ist.

17. Austragvorrichtung nach Anspruch 15, **dadurch gekennzeichnet, dass** der Mediumkanal (14a) einen im wesentlichen sternförmigen Querschnitt aufweist.

## Claims

1. Discharge device for at least one medium with a medium reservoir, a medium pump and an applicator (1, 1a) aligned in the direction of an applicator central axis and in the form of a nose olive in which a medium guide is provided and which has an exit area facing away from the medium reservoir and fitted to a narrow end area for a medium discharge, where at least two exit orifices (4, 4a) are provided at the exit area, **characterized in that** the exit orifices (4, 4a) each have a radially outer wall aligned parallel to the applicator central axis and each have a radially inner wall inclined relative to the applicator central axis.

2. Discharge device according to Claim 1, **characterized in that** the exit orifices (4, 4a) are aligned for medium discharge in at least two different main discharge directions.

3. Discharge device according to Claim 1 or 2, **characterized in that** at least one exit orifice for a main discharge direction is arranged in an angle range (6, 6a) of 10° to 80° relative to the applicator central axis.

4. Discharge device according to one of the preceding claims, **characterized in that** the at least two exit orifices (4, 4a) are arranged over a circumference of the applicator (1, 1a) evenly distributed relative to an applicator central axis (5, 5a).

5. Discharge device according to one of the preceding claims, **characterized in that** a flow guide geometry (15, 16) for the medium is provided in front of at least one exit orifice (4) when seen in the discharge direction.

6. Discharge device according to Claim 5, **characterized in that** a medium channel (14) of a filler section (8) has on an end face facing the exit area conical recess at the end as the flow guide geometry (15, 16).

7. Discharge device according to one of the preceding claims, **characterized in that** at least one spacer element (11) is provided on an inner geometry of the applicator for positioning a filler section (8).

8. Discharge device according to Claim 5, **characterized in that** a guide cone (15) is provided as the flow guide geometry (15, 16) on an inner face (10) of the applicator facing the medium pump.

9. Discharge device according to one of the preceding claims, **characterized in that** a valve device is provided inside the applicator (1, 1a) for at least one exit orifice (4, 4a).

10. Discharge device according to Claim 9, **characterized in that** the valve device can be operated by a control device.

11. Discharge device according to one of the preceding claims, **characterized in that** the at least two exit orifices (4, 4a) are aligned relative to one another such that their main discharge directions intersect.

12. Discharge device according to one of Claims 1 to 5 or 8 to 11, **characterized in that** the applicator (1a) is made as a single-piece plastic component.

13. Discharge device according to one of Claims 1 to 5 or 8 to 12, **characterized in that** at least one medium channel (14a) is integrated in one piece into the applicator (1a).

14. Discharge device according to Claim 13, **characterized in that** the medium channel (14a) has an entry area (17a) connectable to a medium pump and designed such that it provides a tight plug-in connection with an exit connector (19a) of the medium pump in the assembled state.

15. Discharge device according to Claim 13 or 14, **characterized in that** the medium channel (14a) is split into individual channel sections (18a) tapering to the exit orifices (4a) in an exit area facing away from the medium pump.

16. Discharge device according to Claim 13, **characterized in that** the medium channel (14a) is designed with a cross-section substantially constant or evenly tapering in the discharge direction.

17. Discharge device according to Claim 15, **characterized in that** the medium channel (14a) has a substantially star-shaped cross-section.

## Revendications

1. Dispositif de distribution pour au moins un fluide, comprenant un réservoir de fluide, une pompe à fluide et un applicateur (1, 1a), sous forme d'olive nasale, orienté en direction d'un axe médian d'applicateur, dans lequel est prévu un guide de fluide et qui présente une zone de sortie pour une distribution de fluide opposée au réservoir de fluide et située sur une extrémité effilée, au moins deux orifices de sortie (4, 4a) étant prévus dans la zone de sortie, **caractérisé en ce que** chaque orifice de sortie (4, 4a) présente une paroi radialement extérieure, qui est orientée parallèlement à l'axe médian d'applicateur, et une paroi radialement intérieure, qui est inclinée par rapport à l'axe médian d'applicateur.

2. Dispositif de distribution selon la revendication 1, **caractérisé en ce que** les orifices de sortie (4, 4a) pour la distribution de fluide sont orientés selon au moins deux directions principales de distribution différentes.

3. Dispositif de distribution selon la revendication 1 ou 2, **caractérisé en ce qu'**au moins un orifice de sortie pour une direction principale de distribution est situé dans une plage angulaire (6, 6a) de 10° à 80° par rapport à l'axe médian d'applicateur.

4. Dispositif de distribution selon l'une des revendications précédentes, **caractérisé en ce que** les au moins deux orifices de sortie (4, 4a) sont régulièrement répartis sur un pourtour de l'applicateur (1, 1a) autour d'un axe médian d'applicateur (5, 5a).

5. Dispositif de distribution selon l'une des revendications précédentes, **caractérisé en ce que**, vue dans le sens de distribution, est prévue une géométrie directrice de flux (15, 16) pour le fluide en amont d'au moins un orifice de sortie (4).

6. Dispositif de distribution selon la revendication 5, **caractérisé en ce qu'**un conduit de fluide (14) d'une fourrure (8) présente sur une face orientée vers la zone de sortie un évidement facial et conique tenant lieu de géométrie directrice de flux (15, 16).

7. Dispositif de distribution selon l'une des revendications précédentes, **caractérisé en ce que** sur une géométrie intérieure de l'applicateur (1) est prévu au moins un élément intercalaire (11) pour le positionnement d'une fourrure (8).

8. Dispositif de distribution selon la revendication 5, **caractérisé en ce que** sur une surface intérieure (10) de l'applicateur orientée vers la pompe à fluide est prévu un cône de guidage (15) tenant lieu de géométrie directrice de flux (15, 16).

9. Dispositif de distribution selon l'une des revendications précédentes, **caractérisé en ce que** dans l'applicateur (1, 1a) est prévu un dispositif faisant soupape pour au moins un orifice de sortie (4, 4a).

10. Dispositif de distribution selon la revendication 9, **caractérisé en ce que** le dispositif faisant soupape peut être commandé par un dispositif de commande.

11. Dispositif de distribution selon l'une des revendications précédentes, **caractérisé en ce que** les au moins deux orifices de sortie (4, 4a) sont disposés l'un/les uns par rapport à l'autre / aux autres de manière telle que leurs directions principales de distribution se croisent.

12. Dispositif de distribution selon l'une des revendications 1 à 5 ou 8 à 11, **caractérisé en ce que** l'applicateur (1a) est fabriqué comme composant en matière synthétique d'une seule pièce.

13. Dispositif de distribution selon l'une des revendications 1 à 5 ou 8 à 12, **caractérisé en ce que** dans l'applicateur (1a) est intégré au moins un conduit de fluide (14a) en formant une seule pièce.

14. Dispositif de distribution selon la revendication 13, **caractérisé en ce que** le conduit de fluide (14a) présente une zone d'entrée (17a) pouvant être reliée à une pompe à fluide et conçue de manière telle qu'à l'état monté, elle constitue un raccord par emboîtement étanche avec un manchon de sortie (19a) de la pompe à fluide.

15. Dispositif de distribution selon la revendication 13 ou 14, **caractérisé en ce que** dans une zone de sortie opposée à la pompe à fluide, le conduit de fluide (14a) est divisé en différentes parties de conduit (18a) s'étendant en direction des orifices de sortie (4a).

16. Dispositif de distribution selon la revendication 13, **caractérisé en ce que** le conduit de fluide (14a) est formé avec une section constante pour l'essentiel ou décroissant régulièrement dans le sens de distribution.

17. Dispositif de distribution selon la revendication 15, **caractérisé en ce que** le conduit de fluide (14a) présente une section en forme d'étoile pour l'essentiel.
